# EUROPEAN PATENT SPECIFICATION

(11) **EP 2 673 256 B1**
(45) Date of publication and mention of the grant of the patent: **08.04.2015**
(21) Application number: 12702050.1
(22) Date of filing: 07.02.2012
(51) Int. Cl.: C07C 67/307, C07C 69/708, C07C 41/22, C07C 67/287, C07C 41/01

(54) **PROCESS FOR THE MANUFACTURE OF FLUOROMETHOXYMALONIC ACID DERIVATIVES**
VERFAHREN ZUR HERSTELLUNG VON FLUORMETHOXYMALONSÄUREDERIVATEN
PROCÉDÉ DE FABRICATION DE DÉRIVÉS D'ACIDE FLUOROMÉTHOXYMALONIQUE

(30) Priority: 10.02.2011 EP 11153966
(43) Date of publication of application: 18.12.2013
(73) Proprietor: Solvay SA, 1120 Brussels (BE)
(72) Inventor: BRAUN, Max Josef, 30900 Wedemark (DE); CLAASSEN, Uta, 31249 Hohenhameln (DE)
(74) Representative: Mross, Stefan P.M.
(86) International application number: PCT/EP2012/052035
(87) International publication number: WO 2012/107438

(56) References cited:
- EP-A1- 0 901 999
- US-A- 5 705 710

## Description

The present invention which claims priority to European application N° 11153966.4 filed on February 10, 2011 relates to a process for the manufacture of fluoromethoxymalonic acid derivatives and to the use of this process in the manufacture of Sevoflurane, CF₃-CH(OCH₂F)-CF₃. The present invention also relates to the use of the fluoromethoxymalonic acid derivatives prepared according to said process.

Sevoflurane is an important volatile anesthetic agent particularly suited for administration to patients during outpatient surgery. Sevoflurane is known to provide patients with a rapid rate of recovery from the anesthesia. An additional advantage of this anesthetic agent is that it can be used as an induction agent since it is not pungent and allows a rapid and smooth induction without breath holding or laryngospasm as may occur with other inhalation agents. A smooth uneventful induction is especially valuable for pediatric anesthesia where the use of intravenous induction agents can result in numerous problems and is often contraindicated.

Many of the commercial processes for the preparation of Sevoflurane make use of hexafluoroisopropanol (CF₃-CH(OH)-CF₃ (HFIP)) as chemical intermediate and as starting material. In most of these processes, unreacted HFIP may remain in the product mixture and it is well known in the art that HFIP is difficult to remove from the crude Sevoflurane product. The total manufacturing costs are mainly based on the material costs for HFIP manufacturing which is expensive. Several processes have thus been developed to recover HFIP, for instance from the waste stream or by purification of crude Sevoflurane, as exemplified in WO 2004/065340 and WO 2009/085247. Some other methods for the synthesis of Sevoflurane, avoiding the difficult separation of Sevoflurane and HFIP, have also been described, for example in US 5,969,193, US 5,705,710 and US 6,100,434, but said methods are complicated.

More recently, the present inventors have found a process for the manufacture of Sevoflurane comprising fluorination of a substituted malonic acid derivative of formula R₁OOC-CH(CH₂X)-COOR₂ or R₃HNOC-CH(CH₂X)-CONHR₄ which has been described in international patent application EP 2010/061645 (published now as WO 2011/018466) from Solvay Fluor GmbH. This process allows for improved yield, high efficiency and lower manufacturing cost in particular compared to the known processes whereby HFIP is used as chemical intermediate. This international patent application also describes a process for the manufacture of said substituted malonic acid derivative from compounds R₁OOC-CH(Y)-COOR₂ or R₃HNOC-CH(Y)-CONHR₄ with Y being a leaving group which can be substituted by nucleophilic substitution with an O-nucleophile, or from a hydroxyl malonic acid derivative of formula R₁OOC-CH(OH)-COOR₂ or R₃HNOC-CH(OH)-CONHR₄ with a CH₂-electrophile in the presence of a leaving group transfer agent.

In this context, the purpose of the present invention is to provide a process for the manufacture of fluoromethoxy malonic acid compounds starting from compounds of formula R₁OOC-CH(CH₂X)-COOR₂ or R₃HNOC-CH(CH₂X)-CONHR₄.

The present invention therefore relates to a process for the manufacture of compounds of formula (I) or (II)

R₁OOC-CH(OCH₂F)-COOR₂ (I)

R₃HNOC-CH(OCH₂F)-CONHR₄ (II)

wherein
- R₁ and R₂ are equal or different from each other and are independently selected from H ; alkyl groups having from 1 to 10 carbon atoms which are optionally substituted by at least one halogen atom ; aralkyl groups ; and aryl groups,
- R₃ and R₄ are equal to or different from each other and are independently selected from H ; alkyl groups having from 1 to 10 carbon atoms which are optionally substituted by at least one halogen atom ; aralkyl groups ; and aryl groups,
comprising reacting a compound of formula (III) or (IV)

R₁OOC-CH(OCH₂X)-COOR₂ (III)

R₃HNOC-CH(OCH₂X)-CONHR₄ (IV)

wherein
- X is a leaving group that can be substituted by nucleophilic substitution,
- R₁ and R₂ are equal or different from each other and are independently selected from H ; alkyl groups having from 1 to 10 carbon atoms which are optionally substituted by at least one halogen atom ; aralkyl groups ; and aryl groups,
- R₃ and R₄ are equal or different from each other and are independently selected from H ; alkyl groups having from 1 to 10 carbon atoms which are optionally substituted by at least one halogen atom ; aralkyl groups ; and aryl groups,
with at least one source of nucleophilic fluorine.

In the present invention, the compounds of formula (I) or (II) are also referred to as fluoromethoxy malonic acid derivatives.

Advantages of the process include inter alia the fact of being simple, highly efficient and low cost while allowing the preparation of compounds (I) and (II), i.e. fluoromethoxymalonic acid derivatives, starting from compounds of formula (III) or (IV) as described above. This is especially advantageous as the fluoromethoxy compounds are more stable than compounds of formula (III) and (IV) as described above, in particular than the corresponding halogenomethoxy compounds, such as the chloromethoxy compounds. This process has also the advantage to allow the preparation of, for instance, the chloromethoxy derivatives of formula (III) or (IV) according to the processes described in unpublished international patent application EP 2010/061645, said chloromethoxy compounds being easier to prepare than the corresponding fluoromethoxy compounds, the transformation from the chloromethoxy to the fluoromethoxy compound being then easily conducted in a second step according to the present invention, starting from a more stable intermediate.

For the purpose of the present invention, the term "aryl group" refers to an aromatic ring group such as phenyl and naphthyl, and phenyl and naphthyl substituted by at least 1 halogen atom. The term "aralkyl group" refers to an aromatic ring group substituted with alkyl groups such as tolyl, biphenylyl, etc.

In the process of the present invention, the source of nucleophilic fluorine is preferably a fluoride, more preferably a fluoride selected from the group consisting of inorganic fluorides and organic fluorides, especially from metal fluorides and organic fluorides, e.g. onium fluorides, more particularly from alkali fluorides and ammonium fluorides, most preferably from alkali fluorides. Suitable examples of metal fluorides are silver(I)fluoride (AgF) and silver(II)fluoride (AgF₂). Suitable examples of alkali fluorides are sodium fluoride (NaF), potassium fluoride (KF), lithium fluoride (LiF), and cesium fluoride (CsF), advantageously potassium fluoride. Examples of ammonium fluorides are NH₄F and R₄NF where R is an alkyl group such as methyl, ethyl, propyl or butyl.

In the process of the invention, in formulas (I) to (IV), R₁, R₂, R₃ and R₄ may be equal or different from each other and are preferably selected from the group consisting of H, C1-C4 alkyl groups optionally substituted by at least one halogen atom, and aryl groups such as phenyl. R₁, R₂, R₃ and R₄ are more preferably independently from each other selected from the group consisting of H, linear or branched C1-C4 alkyl groups optionally substituted by at least one halogen atom ; particularly preferably from the group consisting of H, methyl, ethyl, n-propyl and isopropyl groups, each optionally substituted by at least one halogen atom ; very preferably from the group consisting of H, methyl and ethyl groups, each optionally substituted by at least one halogen atom. Especially preferably, R₁ and R₂ or R₃ and R₄ are equal; most preferably, R₁ and R₂ are equal and are H or ethyl. R₃ and R₄ are preferably equal and are H or ethyl.

In formulas (III) and (IV) used in the process of the present invention, X may typically be selected from the group consisting of halogens except fluorine ; and oxygen containing functional groups, preferably from bromine, chlorine, iodine, O-tosylate (OTos), O-mesylate (OMes), O-triflate (OTf) and O-trimethylsilyl (OTMS), more preferably from chlorine, OTos, OMes and OTMS, especially preferably chlorine.

In a particularly preferred aspect, R₁ and R₂ are H atoms and X is chlorine, so the compounds of formulas (I) and (III) are respectively the diacids HOOC-CH(OCH₂F)-COOH and HOOC-CH(OCH₂Cl)-COOH.

In another particularly preferred aspect, R₁ and R₂ are ethyl groups and X is chlorine, so the compound of formulas (I) and (III) are respectively the diesters EtOOC-CH(OCH₂F)-COOEt and EtOOC-CH(OCH₂Cl)-COOEt.

The process of the present invention is typically conducted in liquid phase. This process is usually conducted in the presence of a solvent, preferably a polar solvent, more preferably a polar aprotic solvent. Examples of suitable solvents are alkyl nitriles or alkylene dinitriles, e.g. acetonitrile, dialkylsulfoxides, e.g. dimethylsulfoxide (DMSO), dialkylsulfones, e.g. dimethylsulfone (DMSO₂), cyclic sulfones, e.g. sulfolane, formamides, e.g. dimethylformamide (DMF), pyrrolidones, e.g. N-methyl-2-pyrrolidone (NMP), ketones, e.g. acetone, esters, e.g. ethyl acetate, cyclic ethers, e.g. tetrahydrofurane, halogenated carbons which optionally may also comprise hydrogen as substituent or substituents, e.g. dichloromethane, aromatic compounds, e.g. toluene, fluorosubstituted aromatic compounds, e.g. CF₃-toluene, and ionic liquids, e.g. those ionic liquids described in US patent application publication 20090242840. In this paragraph, the term "alkyl" preferably denotes methyl, ethyl, n-propyl or isopropyl ; the term "alkylene" preferably denotes methylene, ethylene or propylene.

In the process of the present invention, the fluoride is usually used in at least a stoechiometric amount, in particular with a molar ratio of fluoride to compounds of formula (III) or (IV) from 4:1 to 1:1, more particularly from 3:1 to 1:1, especially from 2:1 to 1:1, for example around 1.5.

In the process of the present invention, the reaction is in general conducted under atmospheric pressure. The temperature is usually from 40 to 200°C, often from 50 to 150°C, more often from 70 to 160°C. Most often, the reaction is conducted near the reflux temperature, which will thus depend on the nature of the solvent used.

The duration of the reaction of the process of the invention is typically from 15 minutes to 24 hours, in many cases from 30 minutes to 12 hours, for instance from 1 to 8 hours.

In the process of the present invention, the reaction may be performed in an acid, an acidic, neutral or basic medium, preferably in a neutral or basic medium, more preferably in a neutral medium. The medium may be acidified, neutralized or basified by addition of any suitable pH modifying agent known in the art. Acidic pH modifying agents are for example hydrochloric acid, nitric acid, phosphoric acid, sulfuric acid, formic acid, acetic acid, ascorbic acid, citric acid, or tartaric acid. Alkaline pH modifying agents are for example sodium hydroxide, potassium hydroxide, soda ash, ammonia, or triethylamine. The starting material of formula (III) or (IV) may also be acidified, neutralized or basified prior to its use in the process of the present invention, preferably neutralized or basified, more preferably neutralized, using the same pH modifying agents as described above. The starting material may then further be washed, in particular with water or with an organic solvent, and dried prior to its use in the process of the present invention.

According to one embodiment, the process of the present invention may be carried out in the presence of at least one alkali halide other than alkali fluorides, for instance in the presence of an alkali iodide, such as potassium iodide (KI) or cesium iodide (CsI), and preferably in the presence of potassium iodide. The addition of at least one other alkali halide and in particular of an alkali iodide, especially potassium iodide, has the advantage to accelerate the reaction. Indeed, alkali iodides such as KI or CsI dissolve better than KF and dissociate faster (easier) than KF, thus starting the reaction. Such additional alkali halides are usually used in an amount of about 0.01 to 0.5 mol per mol of fluoride, in many cases from 0.05 to 0.2 mol per mol of fluoride, for instance around 0.1 mol per mol of fluoride.

In a further embodiment, the process for the manufacture of the compounds of formula (I) or (II) as defined above may further comprise separating said compounds of formula (I) or (II) from the reaction mixture by a separation method such as distillation, precipitation and/or crystallization, preferably by distillation, in particular by fractional distillation under vacuum.

In another embodiment, the process of the present invention may further comprise recycling of at least part of the reaction medium, in particular of the by-products. Typical by-products, especially suitable for such a recycling, are compounds of formula (V) or (VI) below

R₁OOC-CH(OH)-COOR₂ (V)

R₃HNOC-CH(OH)-CONHR₄ (VI)

wherein R₁, R₂, R₃ and R₄ have the same meaning as defined above. These compounds of formula (V) and (VI) are also referred to as hydroxymalonic acid derivatives in the present invention. A first possibility is for instance the transformation of the hydroxyl group of these compounds of formula (V) or (VI) into leaving groups that can be substituted by nucleophilic substitution, in particular into O-tosylate (OTos), O-mesylate (OMes), O-triflate (OTf) or O-trimethylsilyl (OTMS). This type of transformation may easily be conducted by reaction of the hydroxyl group with respectively p-toluenesulfonic acid, methanesulfonic acid, trifluoromethanesulfonic acid, trimethylsilyl chloride or bis(trimethylsilyl)acetamide. It is also possible to replace the hydroxyl group of these compounds of formula (V) or (VI) with a halogen other than fluorine, in particular bromine, chlorine or iodine, more particularly chlorine. Such reactions are detailed in international patent application EP 2010/061645 (published now as WO 2011/018466). This is especially advantageous as it allows for increasing the final global yield in compounds of formula (I) or (II).

The compounds of formula (I) and (II) prepared according to the present invention may be used as building blocks for the preparation of chemica compounds, in particular for chemical compounds other than Sevoflurane, more particularly for the preparation of biologically active compounds, especially for the preparation of pharmaceutical or agrochemical compounds. Malonic acid and malonate derivatives are indeed often used as building blocks for the preparation of biologically active compounds, for example as described in WO 2010/133724, WO 2010/118992, or WO 2010/109468. In the present invention, the expression "building block" preferably intends to denote a component that fits with at least another block to form a whole, i.e. an organic starting material that reacts with at least another organic compound to form a new compound comprising more carbon atoms than the original organic compound used as starting material. Thus, the present invention provides a process for the manufacture of chemical compounds other than Sevoflurane, especially for the manufacture of biologically active compounds, especially for the preparation of pharmaceutical or agrochemical compounds, comprising a step of providing the compounds of formula (I) or (II) prepared according to the process described above, and further comprising a step of reacting the compounds of formula (I) or (II) with at least one other reactant to provide said chemical compounds other than Sevoflurane, especially for the manufacture of biologically active compounds, especially for the preparation of pharmaceutical or agrochemical compounds.

The compounds of formula (I) and (II) prepared according to the present invention may also be used as foam blowing agents, in particular for the manufacture of plastic foams and especially for polyurethanes foams. Thus, the present invention also provides a process for the manufacture of a foam, especially a plastic foam, and more preferably, a polyurethane (PU) foam, wherein foamable starting material, e.g. a thermoplastic material or a precursor material for a foam, e.g. a polyol composition and a composition comprising a compound having isocyanate groups, is transformed into a foam in the presence of a compound of formula (I) and/or (II) as foam blowing agent.

The compounds of formula (I) and (II) prepared according to the present invention may also be used for the manufacture of Sevoflurane (CF₃-CH(OCH₂F)-CF₃).

In a preferred embodiment, the present invention therefore also relates to a process for the manufacture of Sevoflurane (CF₃-CH(OCH₂F)-CF₃) comprising the steps of :
(a) manufacturing a compound of formula (I) or (II) according to the process as defined above, and
(b) fluorinating said compound of formula (I) or (II).

This process is especially advantageous as compounds of formula (I) and (II), i.e. fluoromethoxy compounds, are more stable than the corresponding compounds of formula (III) and (IV), and in particular than the other halogenomethoxy compounds, especially than chloromethoxy compounds.

In general, the fluorination reaction is performed in the liquid phase.

In this embodiment, typically, the fluorination reaction is carried out with fluorinating agents. Said fluorinating agents preferably allow fluorinating a halogen, a hydroxyl group, a carboxyl group and other oxygen containing functional groups, in particular substituting carbon-oxygen functionality with carbon-fluorine groups. Typical examples of said fluorinating agents are for example sulfur tetrafluoride (SF₄) ; diethylaminosulfur trifluoride (DAST), dimethylaminosulfur trifluoride (DMAST), 4-tert-butyl-2,6-dimethylphenylsulfur trifluoride (Fluolead^{™}) and bis(2-methoxyethyl)aminosulfur trifluoride (Deoxofluor), SF₄ being the most preferred fluorinating agent. The production of SF₄ can be realized according to the procedure described in the GB Pat. No. 1374054.

According to one specific embodiment, the fluorination with SF₄ is carried out in the presence of anhydrous HF. HF is often used in an amount of about 1 to about 1000 moles, and preferably about 1 to about 500 moles, per mole of SF₄.

In this embodiment, the fluorination can be carried out in the presence or in the absence of a solvent. Suitable solvents may for instance be selected from aromatic hydrocarbons such as benzene, toluene or xylene ; aliphatic hydrocarbons such as pentane or hexane ; halogenated hydrocarbons such as methylene chloride, chloroform, ethylene dichloride ; hydrofluorocarbons such as 1,1,1,3,3-pentafluorobutane (Solkane^{®}365 mfc) ; perfluorocarbons such as perfluorocyclohexane ; ethers such as diethyl ether, dibutyl ether or tetrahydrofuran ; and mixtures thereof. Amongst them, the halogenated hydrocarbons are preferred, methylene chloride being most preferred. These solvents may be used alone or in combination as a mixture. If appropriate, the solvent is used usually in an amount of from 50 to 99 % by weight, preferably from 60 to 99 % by weight, more preferably from 75 to 99 % by weight of the solvent relative to the total weight of the reaction medium. If the reaction mixture is a liquid under the reaction conditions, a solvent is not required. If desired, it is also possible to use starting compounds or reaction products c as solvent. Particularly preferred is the use of Sevoflurane as solvent. Preferably, if Sevoflurane is used as a solvent, it is present in the reaction mixture from the start.

The pressure and the temperature of the fluorination reaction are typically selected such that the reaction mixture remains in the liquid phase. For instance, the fluorination reaction may usually be conducted at a temperature equal to or higher than 30°C, preferably equal to or higher than 40°C, more preferably equal to or higher than 50°C. It is generally carried out at a temperature equal to or lower than 90°C, preferably equal to or lower than 80°C, more preferably equal to or lower than 70°C. A temperature ranging from 50 to 70°C is most preferred. The fluorination reaction is generally conducted under pressure, advantageously under a pressure equal to or greater than about 5 bar (abs), preferably equal to or greater than about 10 bar (abs) up to equal to or less than about 25 bar (abs).

Stoichiometrically, the transformation of a C(O) group to a CF₂ group consumes one molecule of SF₄. The transformation of a C-OR₁ or C-OR₂ group to a C-F group consumes half a molecule of SF₄, as well as the transformation of a OCH₂X group to form a OCH₂F group. Consequently, in the fluorination step according to this embodiment, the range of the molar ratio of compound of formula (I) or (II) to SF₄ is preferably from 1:3 to 1:5, particularly preferably from 1:3.5 to 1:4.5.

In an especially preferred embodiment, the process for the manufacture of Sevoflurane may further comprise purification steps before, between, and/or after steps (a) and (b), preferably at least both between steps (a) and (b) and after step (b).

An advantage of this especially preferred embodiment includes inter alia the possibility to decrease the risk of contamination of the Sevoflurane product by impurities, in particular by halogenated and more particularly by chlorinated impurities. Indeed, if Sevoflurane is prepared by direct fluorination of a chloromethoxy derivative, chlorinated impurities may be present in the resulting product. On the contrary, if the chloromethoxy derivative is first transformed into corresponding fluoromethoxy compound of formula (I) or (II) before fluorination into Sevoflurane, with an intermediate purification step, chlorine will be removed one step earlier and the final product will be less contaminated. The presence of an additional purification step will improve the removal of chlorinated impurities.

The purification steps according to this especially preferred embodiment may typically be separation of respectively the compound of formula (I) or (II) and/or of the Sevoflurane from the reaction mixture by a separation method such as distillation, precipitation and/or crystallization, preferably by distillation, in particular by fractional distillation under vacuum.

In the process for the manufacture of Sevoflurane, the compound of formula (I) or (II) prepared in step (a), prior to the fluorination reaction, is preferably the diacid HOOC-CH(OCH₂F)-COOH or a diester of formula R₁OOC-CH(OCH₂F)-COOR₂ wherein R₁ and R₂ are equal or different from each other and are independently selected from an alkyl group having 1 to 10 carbon atoms which is optionally substituted by at least one halogen atom, an aralkyl group or an aryl group, in particular a diester of formula (I) wherein R₁ and R₂ are ethyl groups, i.e. EtOOC-CH(OCH₂F)-COOEt.

In one embodiment, the compound of formula (I) or (II) prepared in step (a) is the diacid HOOC-CH(OCH₂F)-COOH or the diamide H₂NOC-CH(OCH₂F)-CONH₂, preferably the diacid HOOC-CH(OCH₂F)-COOH.

In a further preferred embodiment, the present invention relates to a process for the manufacture of Sevoflurane (CF₃-CH(OCH₂F)-CF₃) comprising the steps of :
(a) manufacturing a compound of formula (I) or (II)

   R₁OOC-CH(OCH₂F)-COOR₂ (I)

   R₃HNOC-CH(OCH₂F)-CONHR₄ (II)

   wherein
   - R₁ and R₂ are equal or different from each other and are independently selected from alkyl groups having from 1 to 10 carbon atoms which are optionally substituted by at least one halogen atom ; aralkyl groups ; and aryl groups ; preferably, R₁ and R₂ are ethyl groups,
   - R₃ and R₄ are equal to or different from each other and are independently selected from alkyl groups having from 1 to 10 carbon atoms which are optionally substituted by at least one halogen atom ; aralkyl groups ; and aryl groups,
   i.e. a fluoromethoxymalonic acid diester or diamide, according to the process as defined in the present invention,
(b) hydrolyzing the diester or diamide of step (a) into the corresponding diacid HOOC-CH(OCH₂F)-COOH, and
(c) fluorinating said diacid.

This process has the advantage that, first, the fluoromethoxymalonic acid diester (or diamide) may be formed according to the process of the present invention, this product having a good stability, then it can easily be transformed into the corresponding diacid before fluorination step, the fluorination of diacids being more common than the fluorination of diesters. It is preferred to manufacture a diester in step (a). This process could thus be especially advantageous for the manufacture of Sevoflurane at industrial scale.

In this further preferred embodiment, the hydrolysis reaction of step (b) of the diester or diamide into the corresponding diacid may be conducted in the presence of water, in any conditions known in the art for the hydrolysis of esters or amides, or by transesterification with organic acids, in particular with aliphatic acids such as for instance acetic acid or trifluoroacetic acid, or even with aromatic carboxylic acids. Said hydrolysis reaction may be catalyzed by acids or bases, especially in the embodiment using water. Typical acids are for instance dilute inorganic acids such as dilute HCl or H₂SO₄, or organic acids such as p-toluenesulfonic acid (pTSA) or small amounts of trifluoromethanesulfonic acid (or triflic acid). Typical bases are for example dilute inorganic bases such as NaOH.

Said hydrolysis reaction is usually conducted at atmospheric pressure or under vacuum and under heating, for instance at a temperature from 40 to 100°C, typically at reflux temperature.

In this further preferred embodiment, the fluorination step (c) may be conducted in the same conditions as described above.

In a further specific embodiment, the processes for the manufacture of Sevoflurane may further comprise separating Sevoflurane from the reaction mixture which is obtained in accordance with any of the preferred embodiments disclosed herein, by a separation method such as distillation, precipitation and/or crystallization, preferably by distillation, in particular by a fractional distillation.

The processes of the present invention and of the preferred embodiments may be carried out batch-wise or continuously. Said processes may be conducted in any suitable reactor such as in an autoclave.

The present invention is further illustrated below without limiting the scope thereto.

### Example 1 : preparation of diethyl-2-(fluoromethoxy)malonate from acidic diethyl-2-(chloromethoxy)malonate

18 g (0.08 mol) of diethyl-2-(chloromethoxy)malonate having a pH of 1 (acidity coming from the hydrolysis of SOCl₂ to H₂SO₄ during aqueous work-up after its manufacturing reaction, i.e. chloromethoxylation) from production, 55 ml of acetonitrile, 7 g of KF (0.12 mol) and 2 g of KI (0.012 mol) were placed in a 100 ml three-necked Teflon^{®} flask fitted with a reflux condenser. The mixture was brought to the boil and refluxed under N₂-atmosphere.

The reaction was followed by gas chromatography (GC) after filtration of the reaction medium samples through syringe filters to remove all solids before injection into the gas chromatograph.

After approximately 6 hours, the GC analysis showed that the starting material had reacted nearly quantitatively, forming about 55 wt % of diethyl-2-(fluoromethoxy)malonate and about 37 wt % of hydroxymalonic ester as a by-product. These compounds were characterized by gas chromatography coupled with mass spectra (GC-MS) and nuclear magnetic resonance (NMR).

The product was purified by evaporating the solvent with a rotary evaporator, followed by fractionation under vacuum. Its ¹³C NMR spectra, measured at 126 MHz in deuterated chloroform shows the following peaks (ppm) : 14.0 (q, 2 C), 62.5 (t, 2 C), 76.8 (d, 1 C), 102.3 (t, J=220.6 Hz, 1 C), 165.4 (s, 2 C). Its ¹⁹F NMR spectra, measured at 471 MHz in deuterated chloroform, shows the following peaks (ppm) : -154.3 (t, J=54.9 Hz, 1 F). The ¹H-NMR as well as MS-spectra also confirmed the product structure.

### Example 2 : Preparation of diethyl-2-(fluoromethoxy)malonate from previously neutralized diethyl-2-(chloromethoxy)malonate

Example 2 was conducted in the same conditions as Example 1, except that the still acidic diethyl-2-(chloromethoxy)malonate from production was washed once with a 10 wt % NaOH solution then with water and then dried with Na₂SO₄ before being engaged in the reaction.

After approximately 2 hours, the GC analysis showed that the starting material had reacted almost quantitatively, forming about 78 wt % of diethyl-2-(fluoromethoxy)malonate and about 22 wt % of hydroxymalonic ester as a by-product.

### Example 3 : Preparation of diethyl-2-(fluoromethoxy)malonate from acidic diethyl-2-(chloromethoxy)malonate in the presence of NEt₃

Example 3 was conducted in the same conditions as Example 1, except that 4 ml of NEt₃ were added twice to the reaction mixture, first after the reaction mixture had reached reflux conditions, to speed up the reaction, and second after 1 hour.

Similar results as in Example 2 were obtained.

## Claims

1. A process for the manufacture of compounds of formula (I) or (II)
R₁OOC-CH(OCH₂F)-COOR₂ (I)
R₃HNOC-CH(OCH₂F)-CONHR₄ (II)
wherein
- R₁ and R₂ are equal or different from each other and are independently selected from H ; alkyl groups having from 1 to 10 carbon atoms which are optionally substituted by at least one halogen atom ; aralkyl groups ; and aryl groups,
- R₃ and R₄ are equal or different from each other and are independently selected from H ; alkyl groups having from 1 to 10 carbon atoms which are optionally substituted by at least one halogen atom ; aralkyl groups ; and aryl groups,
comprising reacting a compound of formula (III) or (IV)
R₁OOC-CH(OCH₂X)-COOR₂ (III)
R₃HNOC-CH(OCH₂X)-CONHR₄ (IV)
wherein
- X is a leaving group that can be substituted by nucleophilic substitution,
- R₁ and R₂ are equal or different from each other and are independently selected from H ; alkyl groups having from 1 to 10 carbon atoms which are optionally substituted by at least one halogen atom ; aralkyl groups, and aryl groups,
- R₃ and R₄ are equal or different from each other and are independently selected from H ; alkyl groups having from 1 to 10 carbon atoms which are optionally substituted by at least one halogen atom ; aralkyl groups ; and aryl groups,
with at least one source of nucleophilic fluorine.

2. The process according to claim 1, wherein X is selected from the group consisting of halogens except fluorine ; and oxygen containing functional groups, preferably from bromine, chlorine, iodine, O-tosylate (OTos), O-mesylate (OMes), O-triflate (OTf) and O-trimethylsilyl (OTMS), more preferably from chlorine, OTos, OMes and OTMS, most preferably chlorine.

3. The process according to claim 1 or 2, wherein respectively R₁ and R₂ or R₃ and R₄ are equal and are selected from H, a methyl group, an ethyl group, a n-propyl group or an isopropyl group, preferably from H or an ethyl group.

4. The process according to anyone of claims 1 to 3, wherein the source of nucleophilic fluorine is a fluoride, preferably selected from the group consisting of inorganic fluorides and organic fluorides, especially from metal fluorides and organic fluorides, more particularly from alkali fluorides and ammonium fluorides, most preferably from alkali fluorides, especially potassium fluoride.

5. The process according to anyone of claims 1 to 4, wherein the reaction is conducted in the presence of a solvent, preferably a polar solvent, more preferably a polar aprotic solvent.

6. The process according to anyone of claims 1 to 5, wherein the reaction is conducted in the presence of at least one alkali halide other than alkali fluorides, in particular in the presence of an alkali iodide, more particularly of potassium iodide or cesium iodide, most particularly of potassium iodide.

7. The process according to anyone of claims 1 to 6, wherein the reaction is performed in an acidic, neutral or basic medium, preferably in a neutral or basic medium, more preferably in a neutral medium.

8. The process according to anyone of claims 1 to 7, further comprising separating the compound of formula (I) or (II) from the reaction mixture, preferably by distillation, in particular by fractional distillation under vacuum.

9. The use of a compound of formula (I) or (II) prepared according to anyone of claims 1 to 8 as a building block for the preparation of biologically active compounds, in particular for the preparation of pharmaceutical or agrochemical compounds.

10. The use of the compound of formula (I) or (II) prepared according to anyone of claims 1 to 8 as a foam blowing agent.

11. A process for the manufacture of Sevoflurane (CF₃-CH(OCH₂F)-CF₃) comprising the steps of:
(a) manufacturing a compound of formula (I) or (II) according to the process of anyone of claims 1 to 8, and
(b) fluorinating said compound of formula (I) or (II).

12. The process according to claim 11, wherein the fluorinating agent is SF₄.

13. The process according to claim 11 or 12, wherein the compound of formula (I) or (II) prepared in step (a) is the diacid HOOC-CH(OCH₂F)-COOH or the diamide H₂NOC-CH(OCH₂F)-CONH₂, preferably the diacid HOOC-CH(OCH₂F)-COOH.

14. The process according to claim 11 or 12, wherein the compound of formula (I) or (II) prepared in step (a) is a diester of formula R₁OOC-CH(OCH₂F)-COOR₂ or a diamide of formula R₃HNOC-CH(OCH₂F)-CONHR₄, wherein
- R₁ and R₂ are equal or different from each other and are independently selected from alkyl groups having 1 to 10 carbon atoms which are optionally substituted by at least one halogen atom, aralkyl groups and aryl groups,
- R₃ and R₄ are equal or different from each other and are independently selected from alkyl groups having 1 to 10 carbon atoms which are optionally substituted by at least one halogen atom, aralkyl groups and aryl groups,
and wherein said diester or diamide is hydrolyzed into the corresponding diacid HOOC-CH(OCH₂F)-COOH prior to fluorination step (b).

15. The process according to claim 14, wherein the compound of formula (I) or (II) prepared in step (a) is a diester of formula R₁OOC-CH(OCH₂F)-COOR₂, wherein R₁ and R₂ are equal or different from each other and are independently selected from alkyl groups having 1 to 10 carbon atoms which are optionally substituted by at least one halogen atom ; aralkyl groups and aryl groups, preferably wherein R₁ and R₂ are ethyl groups.

## Patentansprüche

1. Verfahren zur Herstellung von Verbindungen der Formel (I) oder (II)
R₁OOC-CH(OCH₂F)-COOR₂ (I)
R₃HNOC-CH(OCH₂F)-CONHR₄ (II)
worin
- R₁ und R₂ gleich oder voneinander verschieden sind und unabhängig aus H, Alkylgruppen mit 1 bis 10 Kohlenstoffatomen, die gegebenenfalls durch mindestens ein Halogenatom substituiert sind, Aralkylgruppen und Arylgruppen ausgewählt sind,
- R₃ und R₄ gleich oder voneinander verschieden sind und unabhängig aus H, Alkylgruppen mit 1 bis 10 Kohlenstoffatomen, die gegebenenfalls durch mindestens ein Halogenatom substituiert sind, Aralkylgruppen und Arylgruppen ausgewählt sind,
bei dem man eine Verbindung der Formel (III) oder (IV)
R₁OOC-CH(OCH₂X)-COOR₂ (III)
R₃HNOC-CH(OCH₂X)-CONHR₄ (IV)
worin
- X für eine Abgangsgruppe, die durch nucleophile Substitution substituiert werden kann,
- R₁ und R₂ gleich oder voneinander verschieden sind und unabhängig aus H, Alkylgruppen mit 1 bis 10 Kohlenstoffatomen, die gegebenenfalls durch mindestens ein Halogenatom substituiert sind, Aralkylgruppen und Arylgruppen ausgewählt sind,
- R₃ und R₄ gleich oder voneinander verschieden sind und unabhängig aus H, Alkylgruppen mit 1 bis 10 Kohlenstoffatomen, die gegebenenfalls durch mindestens ein Halogenatom substituiert sind, Aralkylgruppen und Arylgruppen ausgewählt sind,
mit mindestens einer Quelle von nucleophilem Fluor umsetzt.

2. Verfahren nach Anspruch 1, bei dem X aus der Gruppe bestehend aus Halogenen außer Fluor und sauerstoffhaltigen funktionellen Gruppen, vorzugsweise aus Brom, Chlor, Iod, O-Tosylat (OTos), 0-Mesylat (OMes), O-Triflat (OTf) und O-Trimethylsilyl (OTMS), bevorzugter aus Chlor, OTos, OMes und OTMS, ganz besonders bevorzugt Chlor, ausgewählt ist.

3. Verfahren nach Anspruch 1 oder 2, bei dem R₁ und R₂ oder R₃ und R₄ gleich sind und aus H, einer Methylgruppe, einer Ethylgruppe, einer n-Propylgruppe oder einer Isopropylgruppe, vorzugsweise aus H oder einer Ethylgruppe, ausgewählt sind.

4. Verfahren nach einem der Ansprüche 1 bis 3, bei dem es sich bei der Quelle von nucleophilem Fluor um ein Fluorid handelt, das vorzugsweise aus der Gruppe bestehend aus anorganischen Fluoriden und organischen Fluoriden, insbesondere aus Metallfluoriden und organischen Fluoriden, spezieller aus Alkalifluoriden und Ammoniumfluoriden, ganz besonders bevorzugt aus Alkalifluoriden, insbesondere Kaliumfluorid, ausgewählt ist.

5. Verfahren nach einem der Ansprüche 1 bis 4, bei dem die Umsetzung in Gegenwart eines Lösungsmittels, vorzugsweise eines polaren Lösungsmittels, bevorzugter eines polaren aprotischen Lösungsmittels, durchgeführt wird.

6. Verfahren nach einem der Ansprüche 1 bis 5, bei dem die Umsetzung in Gegenwart mindestens eines Alkalihalogenids, das von Alkalifluoriden verschieden ist, bevorzugt in Gegenwart eines Alkaliiodids, bevorzugter von Kaliumiodid oder Caesiumiodid, ganz besonders bevorzugt von Kaliumiodid, durchgeführt wird.

7. Verfahren nach einem der Ansprüche 1 bis 6, bei dem die Umsetzung in saurem, neutralem oder basischem Medium, vorzugsweise in neutralem oder basischem Medium, bevorzugter in neutralem Medium, durchgeführt wird.

8. Verfahren nach einem der Ansprüche 1 bis 7, bei dem man ferner die Verbindung der Formel (I) oder (II) aus der Reaktionsmischung abtrennt, vorzugsweise durch Destillation, insbesondere durch fraktionierte Destillation unter Vakuum.

9. Verwendung einer nach einem der Ansprüche 1 bis 8 hergestellten Verbindung der Formel (I) oder (II) als Baustein zur Herstellung biologisch aktiver Verbindungen, insbesondere zur Herstellung von pharmazeutischen oder agrochemischen Verbindungen.

10. Verwendung der nach einem der Ansprüche 1 bis 8 hergestellten Verbindung der Formel (I) oder (II) als Schaumstoff-Treibmittel.

11. Verfahren zur Herstellung von Sevofluran (CF₃-CH(OCH₂F)-CF₃), das folgende Schritte umfasst:
(a) Herstellung einer Verbindung der Formel (I) oder (II) gemäß dem Verfahren nach einem der Ansprüche 1 bis 8 und
(b) Fluorieren der Verbindung der Formel (I) oder (II).

12. Verfahren nach Anspruch 11, bei dem es sich bei dem Fluorierungsmittel um SF₄ handelt.

13. Verfahren nach Anspruch 11 oder 12, bei dem es sich bei der in Schritt (a) hergestellten Verbindung der Formel (I) oder (II) um die Disäure HOOC-CH(OCH₂F)-COOH oder das Diamid H₂NOC-CH(OCH₂F)-CONH₂, vorzugsweise die Disäure HOOC-CH(OCH₂F)-COOH, handelt.

14. Verfahren nach Anspruch 11 oder 12, bei dem es sich bei der in Schritt (a) hergestellten Verbindung der Formel (I) oder (II) um einen Diester der Formel R₁OOC-CH(OCH₂F)-COOR₂ oder ein Diamid der Formel R₃HNOC-CH(OCH₂F)-CONHR₄ handelt, worin
- R₁ und R₂ gleich oder voneinander verschieden sind und unabhängig aus Alkylgruppen mit 1 bis 10 Kohlenstoffatomen, die gegebenenfalls durch mindestens ein Halogenatom substituiert sind, Aralkylgruppen und Arylgruppen ausgewählt sind,
- R₃ und R₄ gleich oder voneinander verschieden sind und unabhängig aus Alkylgruppen mit 1 bis 10 Kohlenstoffatomen, die gegebenenfalls durch mindestens ein Halogenatom substituiert sind, Aralkylgruppen und Arylgruppen ausgewählt sind,
und wobei der Diester bzw. das Diamid vor dem Fluorierungsschritt (b) zu der entsprechenden Disäure HOOC-CH(OCH₂F)-COOH hydrolysiert wird.

15. Verfahren nach Anspruch 14, bei dem es sich bei der in Schritt (a) hergestellten Verbindung der Formel (I) oder (II) um einen Diester der Formel R₁OOC-CH(OCH₂F)-COOR₂ handelt, worin R₁ und R₂ gleich oder voneinander verschieden sind und unabhängig aus Alkylgruppen mit 1 bis 10 Kohlenstoffatomen, die gegebenenfalls durch mindestens ein Halogenatom substituiert sind, Aralkylgruppen und Arylgruppen ausgewählt sind und worin R₁ und R₂ vorzugsweise für Ethylgruppen stehen.

## Revendications

1. Procédé de fabrication de composés de formule (I) ou (II)
R₁OOC-CH(OCH₂F)-COOR₂ (I)
R₃HNOC-CH(OCH₂F)-CONHR₄ (II)
dans lesquelles
- R₁ et R₂ sont identiques ou différents l'un de l'autre et sont indépendamment choisis parmi H ; les groupes alkyle ayant de 1 à 10 atomes de carbone qui sont optionnellement substitués par au moins un atome d'halogène ; les groupes aralkyle ; et les groupes aryle,
- R₃ et R₄ sont identiques ou différents l'un de l'autre et sont indépendamment choisis parmi H ; les groupes alkyle ayant de 1 à 10 atomes de carbone qui sont optionnellement substitués par au moins un atome d'halogène ; les groupes aralkyle ; et les groupes aryle, comprenant la réaction d'un composé de formule (III) ou (IV)
R₁OOC-CH(OCH₂X)-COOR₂ (III)
R₃HNOC-CH(OCH₂X)-CONHR₄ (IV)
dans lesquelles
- X est un groupe partant qui peut être substitué par substitution nucléophile,
- R₁ et R₂ sont identiques ou différents l'un de l'autre et sont indépendamment choisis parmi H ; les groupes alkyle ayant de 1 à 10 atomes de carbone qui sont optionnellement substitués par au moins un atome d'halogène ; les groupes aralkyle ; et les groupes aryle,
- R₃ et R₄ sont identiques ou différents l'un de l'autre et sont indépendamment choisis parmi H ; les groupes alkyle ayant de 1 à 10 atomes de carbone qui sont optionnellement substitués par au moins un atome d'halogène ; les groupes aralkyle ; et les groupes aryle, avec au moins une source de fluor nucléophile.

2. Procédé selon la revendication 1, dans lequel X est choisi dans le groupe constitué par les halogènes à l'exception du fluor ; et les groupes fonctionnels contenant de l'oxygène, de préférence parmi le brome, le chlore, l'iode, l'O-tosylate (OTos), l'O-mésylate (OMes), l'O-triflate (OTf) et l'O-triméthylsilyle (OTMS), mieux encore parmi le chlore, l'OTos, l'OMes et l'OTMS, idéalement le chlore.

3. Procédé selon la revendication 1 ou 2, dans lequel respectivement R₁ et R₂ ou R₃ et R₄ sont identiques et sont choisis entre H, un groupe méthyle, un groupe éthyle, un groupe n-propyle ou un groupe isopropyle, de préférence entre H ou un groupe éthyle.

4. Procédé selon l'une quelconque des revendications 1 à 3, dans lequel la source de fluor nucléophile est un fluorure, de préférence choisi dans le groupe constitué par les fluorures inorganiques et les fluorures organiques, en particulier parmi les fluorures métalliques et les fluorures organiques, plus particulièrement parmi les fluorures alcalins et les fluorures d'ammonium, idéalement parmi les fluorures alcalins, en particulier le fluorure de potassium.

5. Procédé selon l'une quelconque des revendications 1 à 4, dans lequel la réaction est conduite en présence d'un solvant, de préférence un solvant polaire, mieux encore un solvant polaire aprotique.

6. Procédé selon l'une quelconque des revendications 1 à 5, dans lequel la réaction est conduite en présence d'au moins un halogénure alcalin à part les fluorures alcalins, en particulier en présence d'un iodure alcalin, plus particulièrement d'iodure de potassium ou d'iodure de césium, idéalement d'iodure de potassium.

7. Procédé selon l'une quelconque des revendications 1 à 6, dans lequel la réaction est effectuée dans un milieu acide, neutre ou basique, de préférence dans un milieu neutre ou basique, mieux encore dans un milieu neutre.

8. Procédé selon l'une quelconque des revendications 1 à 7, comprenant en outre la séparation du composé de formule (I) ou (II) du mélange réactionnel, de préférence par distillation, en particulier par distillation fractionnée sous vide.

9. Utilisation d'un composé de formule (I) ou (II) préparé selon l'une quelconque des revendications 1 à 8 comme élément structural pour la préparation de composés biologiquement actifs, en particulier pour la préparation de composés pharmaceutiques ou agrochimiques.

10. Utilisation du composé de formule (I) ou (II) préparé selon l'une quelconque des revendications 1 à 8 comme agent d'expansion de mousse.

11. Procédé de fabrication de sévoflurane (CF₃-CH(OCH₂F)-CF₃) comprenant les étapes consistant à :
(a) fabriquer un composé de formule (I) ou (II) selon le procédé de l'une quelconque des revendications 1 à 8, et
(b) fluorer ledit composé de formule (I) ou (II).

12. Procédé selon la revendication 11, dans lequel l'agent fluorant est SF₄.

13. Procédé selon la revendication 11 ou 12, dans lequel le composé de formule (I) ou (II) préparé à l'étape (a) est le diacide HOOC-CH(OCH₂F)-COOH ou le diamide H₂NOC-CH(OCH₂F)-CONH₂, de préférence le diacide HOOC-CH(OCH₂F)-COOH.

14. Procédé selon la revendication 11 ou 12, dans lequel le composé de formule (I) ou (II) préparé à l'étape (a) est un diester de formule R₁OOC-CH(OCH₂F)-COOR₂ ou un diamide de formule R₃HNOC-CH(OCH₂F)-CONHR₄, dans lesquelles
- R₁ et R₂ sont identiques ou différents l'un de l'autre et sont indépendamment choisis parmi les groupes alkyle ayant 1 à 10 atomes de carbone qui sont optionnellement substitués par au moins un atome d'halogène, les groupes aralkyle et les groupes aryle,
- R₃ et R₄ sont identiques ou différents l'un de l'autre et sont indépendamment choisis parmi les groupes alkyle ayant 1 à 10 atomes de carbone qui sont optionnellement substitués par au moins un atome d'halogène, les groupes aralkyle et les groupes aryle,
et dans lequel ledit diester ou diamide est hydrolysé en diacide correspondant HOOC-CH(OCH₂F)-COOH avant l'étape de fluoration (b).

15. Procédé selon la revendication 14, dans lequel le composé de formule (I) ou (II) préparé à l'étape (a) est un diester de formule R₁OOC-CH(OCH₂F)-COOR₂, dans laquelle R₁ et R₂ sont identiques ou différents l'un de l'autre et sont indépendamment choisis parmi les groupes alkyle ayant 1 à 10 atomes de carbone qui sont optionnellement substitués par au moins un atome d'halogène, les groupes aralkyle et les groupes aryle, de préférence dans laquelle R₁ et R₂ sont des groupes éthyle.
